**Europäisches Patentamt**

**European Patent Office**

**Office européen des brevets**

(11) Veröffentlichungsnummer : **0 350 741 B1**

(12) # EUROPÄISCHE PATENTSCHRIFT

(45) Veröffentlichungstag der Patentschrift :
**13.05.92 Patentblatt 92/20**

(51) Int. Cl.⁵ : **C07C 59/105**, C07C 51/31, C07C 51/235

(21) Anmeldenummer : **89112060.2**

(22) Anmeldetag : **01.07.89**

(54) **Verfahren zur Herstellung von Gluconsäure durch katalytische Oxidation von Glucose.**

(30) Priorität : **09.07.88 DE 3823301**

(43) Veröffentlichungstag der Anmeldung :
**17.01.90 Patentblatt 90/03**

(45) Bekanntmachung des Hinweises auf die
Patenterteilung :
**13.05.92 Patentblatt 92/20**

(84) Benannte Vertragsstaaten :
**AT BE CH DE ES FR GB GR IT LI LU NL SE**

(56) Entgegenhaltungen :
**EP-A- 0 232 202**
**EP-A- 0 233 816**
**DE-A- 1 037 441**
**PATENT ABSTRACTS OF JAPAN, Band 9, Nr. 233 (C-304)[1956], 19. September 1985; & JP-A-60 92 239**

(73) Patentinhaber : **Degussa Aktiengesellschaft**
**Weissfrauenstrasse 9**
**W-6000 Frankfurt am Main 1 (DE)**

(72) Erfinder : **Deller, Klaus, Dr.**
**Friedhofstrasse 47**
**W-6452 Hainburg (DE)**
Erfinder : **Krause, Helmfried**
**Odenwaldstrasse 39**
**W-6458 Rodenbach (DE)**
Erfinder : **Peldszus, Erik**
**Heinrich-Hofmann-Strasse 3**
**W-6467 Hasselroth (DE)**
Erfinder : **Despeyroux, Bertrand, Dr.**
**Kleinfeller Strasse 3**
**W-6450 Hanau 1 (DE)**

EP 0 350 741 B1

## Beschreibung

Die Erfindung beschreibt ein Verfahren zur Herstellung von Gluconsäure bzw. von deren Alkalimetallsalzen durch Oxidation von Glucose mit Sauerstoff oder einem sauerstoffhaltigen Gas in einer wäßrigen alkalischen Lösung in Gegenwart eines ein Platingruppenmetall und Wismut enthaltenden Trägerkatalysators.

Zur Herstellung von Gluconsäure werden neben großtechnisch ausgeübten fermentativen Verfahren auch katalytische Verfahren, welche von Glucose ausgehen und diese mit einem sauerstoffhaltigen Gas in einer wäßrigen alkalischen Lösung in Anwesenheit eines heterogenen Katalysators oxidieren, beschrieben.

In der EP-OS 0 142 725 wird ein solches katalytisches Verfahren beschrieben, wobei ein Palladium-Wismut- Katalysator auf Aktivkohle als Träger eingesetzt wird. Das Verfahren liefert einen Glucoseumsatz von 99.8 % und erbringt eine Ausbeute an Gluconsäure-Natriumsalz von 99.5 %. Die Selektivität für Gluconsäure-Natriumsalz wird mit 99.7 % angegeben; der Anteil an dem Isomerisierungsprodukt Fruktose soll auf 0.1 % vermindert sein. Die Katalysatoraktivität liegt jedoch höchstens bei 1450 Gramm erhaltenes Produkt/Gramm eingesetztes Pd/h Reaktionszeit. Aufgrund der Meßgenauigkeit der verwendeten HPLC- bzw. Ionenchromatographie-Methode von ± 2 Gew.% sind die obigen Angaben zu Selektivität und Menge des Isomerisierungsprodukts Fruktose zweifelhaft.

Die DE-OS 28 36 327 beschreibt die Oxidation von Arylglykolen mit einem Sauerstoff enthaltenden Gas in wäßrigem alkalischen Milieu zu den entsprechenden $\alpha$-Ketocarbonsäuren unter Verwendung eines Platin-Wismut-Katalysators auf Aktivkohleträger.

Die EP-PS 0 005 779 beschreibt die Oxidation von $\alpha$-Hydroxyarylessigsäuren zu Arylglyoxylsäuren mit einem Sauerstoff enthaltenden Gas in wäßrigem alkalischen Milieu an Platinkatalysatoren bei gleichzeitiger Anwesenheit von Blei und/oder Wismut bzw. deren Verbindungen. Ein ähnliches Verfahren wird in der Japan Kokai Tokkyo Koho JP 56/158733 A 2 angegeben.

Die EP-OS 0 151 498 lehrt schließlich die Herstellung von $\alpha$-Ketogluconsäure aus Glucose an einem Platin-Wismut-Katalysator auf Aktivkohle, wobei aber das Gew.-Verhältnis zwischen Katalysator und Glucose 1,17 : 1 beträgt.

Bei allen bekannten Reaktionssystemen mit einem Platin-Wismut-Katalysator auf Aktivkohle wird also die $\alpha$-Hydroxygruppe zusammen mit einer Alkoholgruppe, einer Aldehydgruppe oder einer Carbonylgruppe oxidiert.

Es konnte nun gefunden werden, daß sich Glucose mit Sauerstoff in alkalischem Milieu selektiv und mit einer gegenüber der Reaktion gemäß EP-OS 0 142 725 stark erhöhten Reaktionsgeschwindigkeit zu Gluconsäure umsetzen läßt, wenn man die Reaktion an einem Platin, Palladium und Wismut enthaltenden Trägerkatalysator mit Aktivkohle als Träger an einer Katalysatormenge durchführt, welche gegenüber der in der EP-OS 0 151 498 Vorgeschlagenen derart herabgesetzt ist, daß nur noch ein Gew.-Verhältnis zwischen Katalysator und Glucose von höchstens 0.2 : 1 vorliegt.

Gegenstand der Erfindung ist demgemäß ein Verfahren zur Herstellung von Gluconsäure bzw. von deren Alkalimetallsalzen durch Oxidation von Glucose mit Sauerstoff oder einem sauerstoffhaltigen Gas in einer wäßrigen alkalischen Lösung in Gegenwart eines ein Platingruppenmetall und Wismut enthaltenden Trägerkatalysators, welches dadurch gekennzeichnet ist, daß man die Reaktion bei einer Temperatur von 20 - 80°C und bei einem durch laufende Zugabe von Alkalimetallhydroxid aufrechterhaltenen pH-Wert von 8,0 - 11 bis zum Erreichen eines durch Zwischenanalyse erkennbaren optimalen Umsetzungsgrades an einem Trägerkatalysator durchführt, dessen Menge zur Glucose in einem Gewichtsverhältnis von höchstens 0,2 steht, eine auf Aktivkohle als Träger aufgebrachte, aus Platin, Palladium und Wismut bestehende aktive Phase aufweist und 0,1 bis 10 Gew.% der Platin und Palladium enthaltenden Edelmetallkomponente und 0,01 bis 20 Gew.% Wismut bei einem Gewichtsverhältnis zwischen Platin und Palladium von 1 : 99 bis 70 : 30, vorzugsweise 15 : 85 bis 50 : 50 und insbesondere 15 : 85 bis 25 : 75, enthält und nach Abtrennung des Katalysators das gebildete Alkalimetallgluconat gegebenenfalls in die freie Säure überführt.

Der Katalysator ist erhältlich durch Imprägnieren bzw. Belegen des Trägers mit Salzen, Oxiden oder Hydroxiden der genannten Platingruppenmetalle und des Wismuts und Reduzieren.

Für die Einbringung der Platingruppenmetalle in den Katalysator können Platinverbindungen, wie $H_2PtCl_6$, $H_2PtCl_4$. Platinnitrat oder Palladiumverbindungen, wie $PdCl_2$, $H_2PdCl_4$ oder Palladiumnitrat in Form von Lösungen bzw. Oxidverbindungen, wie Platinoxidhydrate, PdO oder $Pd(OH)_2$ in Form von Suspensionen in beliebigen Suspensionsmitteln verwendet werden.

Für die Einbringung des Wismuts in den Katalysator können lösliche und unlösliche Wismutverbindungen, wie Wismuttrichlorid, Wismutoxichlorid, Wismuthydroxynitrat und Wismuttrioxid herangezogen werden.

Zur Katalysatorherstellung wird das gleichzeitige Einbringen der gelösten Platingruppenmetall- und Wismutverbindungen in den Träger bevorzugt.

Hierin besteht ein Unterschied zu dem bekannten Pd-Bi-Katalysator auf Aktivkohle gemäß der eingangs

2

besprochenen EP-OS 0 142 725, bei dem obligatorisch zuerst mit Wismut und nachfolgendem längeren Einwirkungsintervall und dann erst mit dem Edelmetall imprägniert werden muß.

Als Reduktionsmittel für die Überführung von durch Imprägnieren oder Abscheiden in den Träger eingeführten Platingruppenmetall- und Wismutverbindungen können Formaldehyd, Hydrazin, Wasserstoff und andere übliche Reduktionsmittel eingesetzt werden. Formaldehyd wird bevorzugt.

Das Verfahren erbringt gegenüber dem mit einem Pd-Bi-Katalysator auf Aktivkohle arbeitenden Verfahren gemäß EP-OS 0 142 727 wesentlich erhöhte Reaktionsgeschwindigkeiten- keiten : Die Aktivitätswerte liegen mit Werten über 4000 g erhaltenes Gluconsäureprodukt/Gramm eingesetztes Gesamtedelmetall/h Reaktionszeit am erreichbaren Optimum.

Die Reaktion kann grundsätzlich bei Drucken des gasförmigen Oxidationsmittels von Normaldruck bis 10 bar durchgeführt werden. Ein Arbeiten bei Normaldruck oder leichtem Überdruck (3,0 bar) wird bevorzugt.

Die Glucose kann als 5 - 50, vorzugsweise 10 - 20 Gew.%ige wäßrige Lösung eingesetzt werden.

Bevorzugt hält man eine Reaktionstemperatur von 30 - 60°C ein. Der zur Neutralisation der gebildeten Gluconsäure benötigte pH-Wert liegt vorzugsweise bei 9 - 11; der pH-Wert wird durch laufenden Zusatz von NaOH zur wäßrigen Lösung eingestellt.

Als Träger wird Aktivkohle in pulvriger Form bevorzugt, obwohl die Verwendung von granuliertem Material nicht ausgeschlossen ist. Aktivkohle mit den folgenden Eigenschaften verleiht dem Katalysator eine hohe Aktivität, wodurch eine niedrige Glucoseisomerisierung und eine hohe Glucoseausbeute ermöglicht werden: BET-Oberfläche nach DIN 66 131 > 500 $m^2$/g, Gesamtporenvolumen > 0.5 ml/g und Aschegehalt < 5 Gew.%.

Für die Erzielung hoher Aktivität und Selektivität ist es günstig, beispielsweise bei Einsatz eines Katalysators mit 1 ,0 Gew.% Pt, 4,0 Gew.% Pd und 5 Gew.% Bi auf Aktivkohle, ebenfalls ein unter 0,1 : 1 liegendes Gewichtsverhältnis zwischen Katalysator und reiner Glucose anzuwenden.

Der nach Erreichen des optimalen Umsatzgrades und Abbruch der Reaktion von der farblosen bis höchstens leicht gelbstichigen Lösung abfiltrierte Katalysator kann in mehreren Ansätzen wiederverwendet werden.

Eine wesentliche Maßnahme im Rahmen des erfindungsgemäßen Verfahrens beruht im Reaktionsabbruch nach Feststellung des optimalen Umsetzungsgrades nach einigen Probenahmen in einem Pilotversuch. Der optimale Umsetzungsgrad ist erreicht, wenn die Glucose z. B. zu mindestens 99 % in Gluconsäure umgewandelt ist. Es konnte festgestellt werden, daß ein Verweilen der Produktlösung in Kontakt zum Katalysator über diesen Zeitpunkt hinaus zur Ansammlung von unerwünschten Neben- bzw. Folgeprodukten, wie Glucarsäure, Weinsäure, Tartronsäure und Oxalsäure führt, welche die Lösung des reinen Gluconats im Laufe der Reaktionszeit immer mehr verunreinigen. Man ermittelt daher durch einen Pilotansatz den optimalen Umsetzungsgrad im Rahmen der Meßgenauigkeit einer chromatographischen Analysenmethode wie der HPLC (High Pressure Liquid Chromatography = Hochdruck-Flüssigkeitschromatographie) oder der Ionenchromatographie und kann dann in den folgenden Ansätzen die ermittelte optimale Reaktionszeit einhalten.

Im Vergleich zum Stand der Technik hat die Erfindung den Vorteil einer sehr rasch verlaufenden und hochselektiven Bildung von reiner Gluconsäure aus Glucose. Die Aktivitätswerte liegen mit mehr als 4000 g erhaltener Gluconsäure/g eingesetzem Gesamt-Edelmetall pro Stunde Reaktionszeit außergewöhnlich hoch. Nicht vorhersehbar war, daß durch die beschriebene Verfahrensführung eine rasche und dabei hochselektive Herstellungsmöglichkeit für Gluconat erschlossen wird, indem weitere Oxidationsreaktionen an der entstandenen Gluconsäure ebenso wie die Isomerisierung der Glucose verhindert werden.

Ebenfalls nicht voraussehbar war, daß durch den Einsatz von Platin oder Platin plus Palladium neben Wismut keine 2-Ketogluconsäure in nennenswerten Mengen in der Reaktionslösung gebildet wird.

Schließlich war auch nicht vorhersehbar, daß ein synergistischer Effekt durch die Verwendung von Palladium neben Platin und Wismut erzielt werden kann.

Die Erfindung wird nachfolgend anhand von Ausführungsbeispielen weiter erläutert.

Beispiel 1

Katalysatorpräparation

90 g einer makroporösen (< 5 nm), pulverförmigen Aktivkohle mit 20 µm mittlerem Teilchendurchmesser werden in 0,8 l destilliertem Wasser suspendiert. 5,57 g $Bi_2O_3$ [entsprechend 5 g Bi] in 10 ml HCl (konz.) gelöst (im weiteren Text als "Wismut-Lösung" bezeichnet), werden mit 4 g einer 25 Gew.%igen wäßrigen Hexachloroplatinsäurelösung (entsprechend 1 g Pt) und mit 20 g einer 20 Gew.%igen wäßrigen Tetrachloropalladiumsäurelösung (entsprechend 4 g Pd) gemischt und zur Kohlesuspension gegeben.

Anschließend wird auf 80° C erwärmt. Durch Zugabe von 13 g NaOH als 10 Gew.%ige wäßrige Lösung wird die Suspension alkalisch gestellt (pH = 10,0). Nach 20 Minuten Rühren bei 80° C wird durch Zugabe von 6 ml wäßriger Formaldehydlösung (37 Gew.%) die Platin-, Palladium und Wismutverbindung reduziert. Unter Rühren wird die Suspension 15 Minuten bei 80° C gehalten und danach abfiltriert und gewaschen. Man erhält einen Pt/Pd/Bi-Aktivkohle-Katalysator mit 1 Gew.% Pt, 4 Gew.% Pd und 5 Gew.% Bi.

Glucoseoxidation

100 ml einer wäßrigen Glucoselösung mit 16 g Glucose (99 Gew.%ig) (entsprechend $8,80 \times 10^{-2}$ Mol) und 0,24 g des oben beschriebenen Katalysators (1,5 Gew.% Katalysator in bezug auf Glucose) werden in einem 250 ml Rührreaktor mit Begasungsrührer, Thermometer, Alkalidosierung, pH-Elektrode und Sauerstoffzufuhr überführt. Bei einer Reaktionstemperatur von 56° C wird der Sauerstoff durch den Begasungsrührer in der Lösung verteilt.

Die Reaktion findet unter Normaldruck statt. Das entstehende Reaktionsprodukt Gluconsäure wird laufend durch Zutropfen von 10 Gew.%iger Natronlauge neutralisiert. Der pH-Wert der Suspension beträgt dabei 10,0.

Nach den in folgender Tabelle angegebenen Reaktionszeiten werden Proben entnommen und davon der Katalysator abfiltriert. Das Filtrat wird mittels Ionenchromatographie und HPCL analysiert. Dabei werden folgende Werte gefunden:

| Zeit [min] | 18 | 20 | 25 | 30 |
|---|---|---|---|---|
| Stoffmenge [Mol $\times 10^{-2}$] | | | | |
| Glucose | 0,15 | < 0,01 | < 0,01 | < 0,01 |
| Gluconsäure | 8,50 | 8,50 | 8,44 | 8,13 |
| Fructose | 0,08 | 0,13 | 0,13 | 0,13 |
| Glucarsäure | 0,03 | 0,05 | 0,09 | 0,32 |
| Weinsäure | < 0,01 | < 0,01 | 0,05 | 0,07 |
| Tartronsäure | < 0,01 | < 0,01 | 0,09 | 0,17 |
| Oxalsäure | < 0,01 | 0,01 | 0,06 | 0,14 |

```
Umsatz (20'):            100 %
Selektivität (20'):       98 %
Aktivität (20'):        4.200 g [Gluconsäure]/g
                              [Gesamtedelmetall] ×
                              Stunde
```

Der Katalysator wird von der Reaktionslösung abgetrennt und das entstandene Reaktionsprodukt Natriumgluconat durch Ionenaustausch in die freie Säure überführt. Der abfiltrierte Katalysator wird erneut (mit gleichem Ergebnis) eingesetzt.

Vergleichsbeispiel 1

Entsprechend dem Beispiel 1 der EP-OS 0 142 725 wird ein Pd-Bi/Aktivkohle-Katalysator mit 5 Gew.% Pd und 5 Gew.% Bi hergestellt und unter folgenden Bedingungen in der Glucoseoxidation getestet:

Temperatur:     50°C
pH:             9,5
Reagenzien:     - 16 g Glucose, 99 Gew.%ig (entsprechend $8,88 \times 10^{-2}$ Mol)
                - 0.24 g Katalysator, entspricht 1,5 Gew.% in bezug auf Glucose
                - 10 Gew.%ige NaOH (zur pH-Einstellung

Die Versuchsauswertung erfolgt wie in Beispiel 1 beschrieben. Es werden folgende Werte ermittelt:

| Zeit [min] | 10 | 15 | 20 | 25 | 45 | 60 |
|---|---|---|---|---|---|---|
| Stoffmenge [Mol x $10^{-2}$] | | | | | | |
| Glucose | 7,00 | 5,53 | 4,07 | 3,09 | 0,04 | 0,02 |
| Gluconsäure | 1,60 | 3,00 | 4,40 | 5,40 | 8,46 | 8,50 |
| Fructose | 0,16 | 0,20 | 0,26 | 0,27 | 0,26 | 0,26 |
| Glucarsäure | < 0,01 | < 0,01 | < 0,01 | < 0,01 | 0,01 | 0,01 |
| Weinsäure | < 0,01 | < 0,01 | < 0,01 | < 0,01 | < 0,01 | < 0,01 |
| Tartronsäure | <0,01 | < 0,01 | < 0,01 | < 0,01 | < 0,01 | < 0,01 |
| Oxalsäure | < 0,01 | < 0,01 | < 0,01 | < 0,01 | < 0,01 | < 0,01 |

Umsatz (45'): 99,5 %

Selektivität (45'): 97 %

Aktivität (45'): 1.800 g [Gluconsäure]/g [Pd] x Stunde

Während hier Umsatz und Selektivität befriedigen, ist die Aktivität unzureichend.

<u>Vergleichsbeispiel 2</u>

Die Katalysatorpräparation erfolgt wie in Beispiel 1 beschrieben, aber mit dem Unterschied, daß eine Mischung aus einer 20 Gew.%igen wäßrigen Tetrachlorpalladiumsäure und Wismutlösung zugegeben wird. Man erhält einen Pd/Bi-Aktivkohle-Katalysator mit 5 Gew.% Pd und 5 Gew.% Bi. Die Glucoseoxidation und Auswertung erfolgt wie in Beispiel 1 beschrieben. Es werden folgende Werte ermittelt :

| Zeit [min] | | 25 | 32 | 35 | 40 |
|---|---|---|---|---|---|
| Stoffmenge [Mol × 10$^{-2}$] | | | | | |
| Glucose | | 2.47 | 0,20 | 0,01 | < 0,01 |
| Gluconsäure | | 6,12 | 8,23 | 8,40 | 8,38 |
| Fructose | | 0,16 | 0,30 | 0,26 | 0,25 |
| Glucarsäure | | 0,01 | 0,03 | 0,05 | 0,07 |
| Weinsäure | < | 0,01 | 0,01 | 0,01 | 0,01 |
| Tartronsäure | < | 0,01 | 0,01 | 0,01 | 0,02 |
| Oxalsäure | < | 0,01 | 0,01 | 0,03 | 0,03 |

Umsatz (35'):        100 %

Selektivität (35'): 96 %

Aktivität (35'):     2.400 g [Gluconsäure]/g [Pd] ×
                                 Stunde

Während hier der Umsatz und die Selektivität befriedigen, ist die Aktivität ebenfalls unzureichend.

Vergleichsbeispiel 3

Die Katalysatorpräparation erfolgt wie in Beispiel 1 beschrieben, aber mit dem Unterschied, daß nur eine 25 Gew.%ige wäßrige Hexachloroplatinsäure zugegeben wird. Man erhält einen Pt/Aktivkohle-Katalysator mit 5 Gew. % Pt. Die Glucoseoxidation und die Auswertung erfolgt wie in Beispiel 1 beschrieben. Es werden folgende Werte ermittelt :

| Zeit [min] | | 10 | 25 | 40 | 60 | 120 |
|---|---|---|---|---|---|---|
| Stoffmenge [Mol × $10^{-2}$] | | | | | | |
| Gluconsäure | | 1,37 | 2,74 | 3,32 | 4,16 | 4,96 |
| Glucarsäure | | 0,01 | 0,04 | 0,10 | 0,12 | 0,22 |
| Weinsäure | < | 0,01 | 0,01 | 0,03 | 0,03 | 0,11 |
| Tartronsäure | < | 0,01 | 0,02 | 0,03 | 0,05 | 0,16 |
| Oxalsäure | < | 0,01 | 0,02 | 0,03 | 0,03 | 0,05 |

Umsatz (120'): 62 %

Selektivität (120'): 90 %

Aktivität (120'): 400 g [Gluconsäure]/g [Pt] × Stunde

Der Umsatz, die Selektivität und die Aktivität sind hier unbefriedigend.

Beispiel 2

Die Katalysatorpräparation erfolgt wie in Beispiel 1 beschrieben mit folgendem Unterschied:

Zur Aktivkohlesuspension wird eine Mischung aus Hexachloroplatinsäure, Tetrachloropalladiumsäure und Wismut-Lösung in solchen Mengen zugegeben, daß man einen Pt/Pd/Bi-Aktivkohle-Katalysator mit 0,1 Gew.% Pt, 4,9 Gew.% Pd und 5 Gew.% Bi erhält. Die Glucoseoxidation und die Auswertung erfolgt wie in Beispiel 1. Es werden folgende Werte ermittelt :

| Zeit [min] | 18 | 20 | 22 | 25 |
|---|---|---|---|---|
| Stoffmenge $[Mol \times 10^{-2}]$ | | | | |
| Glucose | 0,92 | 0,21 | 0,01 | < 0,01 |
| Gluconsäure | 7,68 | 8,33 | 8,51 | 8,47 |
| Fructose | 0,15 | 0,17 | 0,11 | 0,10 |
| Glucarsäure | 0,01 | 0,02 | 0,09 | 0,12 |
| Weinsäure | < 0,01 | < 0,01 | 0,01 | 0,02 |
| Tartronsäure | < 0,01 | < 0,01 | 0,01 | 0,02 |
| Oxalsäure | < 0,01 | 0,03 | 0,03 | 0,04 |

Umsatz (22'): 100 %

Selektivität (22'): 97 %

Aktivität (22'): 3.800 g [Gluconsäure]/g [Gesamt-Edelmetall] x Stunde

## Beispiel 3

Die Katalysatorpräparation erfolgt wie in Beispiel 1 beschrieben mit folgendem Unterschied:

Zur Aktivkohlesuspension wird eine Mischung aus Hexachloroplatinsäure, Tetrachloropalladiumsäure und Wismut-Lösung in solchen Mengen zugegeben, daß man einen Pt/Pd/Bi-Aktivkohle-Katalysator mit 2,5 Gew.% Pt, 2,5 Gew.% Pd und 5 Gew.% Bi erhält. Die Glucoseoxidation und die Auswertung erfolgt wie in Beispiel 1.

| Zeit [min] | | 25 |
|---|---|---|
| Stoffmenge [Mol × $10^{-2}$] | | |
| Glucose | < | 0,01 |
| Gluconsäure | | 8,56 |
| Fructose | | 0,15 |
| Glucarsäure | | 0,08 |
| Weinsäure | < | 0,01 |
| Tartronsäure | | 0,01 |
| Oxalsäure | | 0,01 |

Umsatz (25'): 100 %

Selektivität (25'): 97 %

Aktivität (25'): 3.400 g [Gluconsäure]/g [Gesamt-Edelmetall] × Stunde

Eine Wiederholung des Ansatzes unter denselben Bedingungen, aber unter Durchführung der Glucoseoxidation bei pH 9 erbrachte vergleichbare Ergebnisse.

Beispiel 4

Die Katalysatorzusammensetzung und -präparation entspricht Beispiel 1. Bei der Glucoseoxidation wird die Reaktionstemperatur auf 30° C gesenkt. Die Versuchsdurchführung und die Auswertung erfolgt wie in Beispiel 1 beschrieben. Es werden folgende Werte ermittelt :

9

| Zeit [min] | 15 | 20 | 25 |
|---|---|---|---|
| Stoffmenge $[Mol \times 10^{-2}]$ | | | |
| Glucose | 1,80 | 0,48 | < 0,01 |
| Gluconsäure | 6,83 | 8,13 | 8,55 |
| Fructose | 0,10 | 0,14 | 0,19 |
| Glucarsäure | 0,01 | 0,01 | 0,02 |
| Weinsäure | < 0,01 | < 0,01 | < 0,01 |
| Tartronsäure | < 0,01 | < 0,01 | < 0,01 |
| Oxalsäure | < 0,01 | < 0,01 | < 0,01 |

Umsatz (25'): 100 %

Selektivität (25'): 97 %

Aktivität (25'): 3.400 g [Gluconsäure]/g [Gesamt-Edelmetall] × Stunde

## Beispiel 5

Die Katalysatorzusammensetzung und -präparation entspricht Beispiel 1. Bei der Glucoseoxidation wird der pH-Wert auf 7,5 gehalten. Die Versuchsdurchführung und die Auswertung erfolgt wie in Beispiel 1. Es werden folgende Werte ermittelt :

| Zeit [min] | | 25 | 30 | 45 |
|---|---|---|---|---|
| Stoffmenge $[Mol \times 10^{-2}]$ | | | | |
| Glucose | | 1,00 | 0,76 | 0,45 |
| Gluconsäure | | 7,58 | 7,75 | 8,10 |
| Fructose | | 0,18 | 0,22 | 0,21 |
| Glucarsäure | | 0,02 | 0,03 | 0,05 |
| Weinsäure | < | 0,01 | < 0,01 | < 0,01 |
| Tartronsäure | < | 0,01 | < 0,01 | < 0,01 |
| Oxalsäure | < | 0,01 | < 0,01 | < 0,01 |

Umsatz (45'):  95 %

Selektivität (45'):  97 %

Aktivität (45'):  1.800 g [Gluconsäure]/g [Gesamt-Edelmetall] x Stunde

Vergleichsbeispiel 4

Die Katalysatorpräparation erfolgt wie in Beispiel 1 beschrieben mit folgendem Unterschied :
Zur Aktivkohlesuspension wird eine Mischung aus Hexachloroplatinsäure und Wismut-Lösung in solchen Mengen zugegeben, daß man einen Pt/Bi-Aktivkohle-Katalysator mit 5 Gew.% Pt und 5 Gew.% Bi erhält. Die Glucoseoxidation und Auswertung erfolgt wie in Beispiel 1. Es werden folgende Werte ermittelt:

11

| Zeit [min] | | 20 | 23 | 25 | 30 |
|---|---|---|---|---|---|
| Stoffmenge [Mol × $10^{-2}$] | | | | | |
| Glucose | | 0,64 | 0,59 | 0,38 | 0,15 |
| Gluconsäure | | 7,90 | 7,91 | 8,10 | 8,33 |
| Fructose | | 0,22 | 0,24 | 0,22 | 0,17 |
| Glucarsäure | < | 0,01 | 0,02 | 0,06 | 0,09 |
| Weinsäure | < | 0,01 | < 0,01 | ≦ 0,01 | 0,01 |
| Tartronsäure | < | 0,01 | < 0,01 | ≦ 0,01 | 0,01 |
| Oxalsäure | < | 0,01 | < 0,01 | ≦ 0,01 | 0,03 |

Umsatz (30'):      98 %

Selektivität (30'):      96 %

Aktivität (20'):      3.900 g [Gluconsäure]/g [Pt] × Stunde

Aktivität (30'):      2.700 g [Gluconsäure]/g [Pt] × Stunde

Der Katalysator weist eine hohe Anfangsaktivität auf. Die Glucose ist aber nach einer Reaktionszeit von 30 Min. nicht vollständig umgesetzt.

Vergleichsbeispiel 5

Die Katalysatorpräparation erfolgt wie in Vergleichsbeispiel 4 beschrieben, jedoch mit der halben Platin- und Wismutmenge. Man erhält einen Pt/Bi-Aktivkohle-Katalysator mit 2,5 Gew.% Pt und 2,5 Gew.% Bi. Die Glucoseoxidation und die Auswertung erfolgt wie in Beispiel 1. Es werden folgende Werte ermittelt :

| Zeit [min] | | 10 | 20 | 30 | 40 | 60 |
|---|---|---|---|---|---|---|
| Stoffmenge $[\text{Mol} \times 10^{-2}]$ | | | | | | |
| Glucose | | 4,45 | 2,60 | 1,80 | 1,00 | 0,95 |
| Gluconsäure | | 4,10 | 5,89 | 6,64 | 7,40 | 7,40 |
| Fructose | | 0,20 | 0,21 | 0,21 | 0,23 | 0,22 |
| Glucarsäure | | 0,01 | 0,03 | 0,06 | 0,07 | 0,10 |
| Weinsäure | < | 0,01 < | 0,01 | 0,01 | 0,01 | 0,05 |
| Tartronsäure | < | 0,01 < | 0,01 | 0,02 | 0,03 | 0,07 |
| Oxalsäure | < | 0,01 < | 0,01 < | 0,01 | 0,03 | 0,01 |

Umsatz (60'): 89 %

Selektivität (60'): 94 %

Aktivität (40'): 3.600 g [Gluconsäure]/g [Pt]
x Stunde

Aktivität (60'): 2.400 g [Gluconsäure]/g [Pt]
x Stunde

Der Katalysator weist eine hohe Anfangsaktivität auf. Glucose wird jedoch unvollständig umgesetzt.

Vergleichsbeispiel 6

Die Katalysatorpräparation erfolgt wie in Vergleichsbeispiel 4 beschrieben mit folgendem Unterschied:
Zur Aktivkohlesuspension wird eine Mischung aus Hexachloroplatinsäure und Wismut-Lösung in solchen Mengen zugegeben, daß man einen Pt/Bi-Aktivkohle-Katalysator mit 5 Gew.% Pt und 10 Gew.% Bi erhält. Die Glucoseoxidation und die Auswertung erfolgt wie in Beispiel 1. Es werden folgende Werte ermittelt:

EP 0 350 741 B1

| Zeit [min] | 20 | 25 | 35 | 40 |
|---|---|---|---|---|
| Stoffmenge [Mol x $10^{-2}$] | | | | |
| Glucose | 1,55 | 0,73 | 0,58 | 0,41 |
| Gluconsäure | 6,93 | 7,65 | 7,64 | 7,60 |
| Fructose | 0,18 | 0,21 | 0,19 | 0,22 |
| Glucarsäure | 0,06 | 0,13 | 0,28 | 0,42 |
| Weinsäure | 0,01 | 0,02 | 0,03 | 0,05 |
| Tartronsäure | 0,03 | 0,03 | 0,06 | 0,09 |
| Oxalsäure | 0,03 | 0,03 | 0,06 | 0,08 |

Umsatz (25'): 92 %

Selektivität (25'): 95 %

Aktivität (25'): 3.000 g [Gluconsäure]/g [Pt] x Stunde

Der Umsatz ist unbefriedigend; Glucose wird unvollständig umgesetzt.

**Patentansprüche**

1. Verfahren zur Herstellung von Gluconsäure bzw. von deren Alkalimetallsalzen durch Oxidation von Glucose mit Sauerstoff oder einem sauerstoffhaltigen Gas in einer wäßrigen alkalischen Lösung in Gegenwart eines ein Platingruppenmetall und Wismut enthaltenden Trägerkatalysators,
**dadurch gekennzeichnet**, daß man die Reaktion bei einer Temperatur von 20 - 80° C und bei einem durch laufende Zugabe von Alkalimetallhydroxid aufrechterhaltenen pH-Wert von 8,0 - 11 bis zum Erreichen eines durch Zwischenanalyse erkennbaren optimalen Umsetzungsgrades an einem Trägerkatalysator durchführt, dessen Menge zur Glucose in einem Gewichtsverhältnis von höchstens 0,2 steht, eine auf Aktivkohle als Träger aufgebrachte, aus Platin, Palladium und Wismut bestehende aktive Phase aufweist und 0,1 bis 10 Gew.% der Platin und Palladium enthaltenden Edelmetallkomponente und 0,01 bis 20 Gew.% Wismut bei einem Gewichtsverhältnis zwischen Platin und Palladium von 1 : 99 bis 70 : 30, vorzugsweise 15 : 85 bis 50 : 50 und insbesondere 15 : 85 bis 25 : 75, enthält und nach Abtrennung des Katalysators das gebildete Alkalimetallgluconat gegebenenfalls in die freie Säure überführt.

2. Verfahren nach Anspruch 1,
**dadurch gekennzeichnet**, daß man die Reaktion bei Drucken des gasförmigen Oxidationsmittels von Normaldruck bis 3.0 bar durchführt.

3. Verfahren nach Anspruch 1 oder 2,
**dadurch gekennzeichnet**, daß man die Glucose als 10 - 20 Gew.%ige wäßrige Lösung einsetzt.

4. Verfahren nach einem der Ansprüche 1 bis 3,
**dadurch gekennzeichnet**, daß man eine Reaktionstemperatur von 30 - 60° C einhält.

5. Verfahren nach einem der Ansprüche 1 bis 4,
**dadurch gekennzeichnet**, daß man bei einem pH-Wert von 9 - 11 arbeitet.

6. Verfahren nach einem der Ansprüche 1 bis 5,
**dadurch gekennzeichnet**, daß man als Katalysatorträger pulverförmige Aktivkohle einsetzt.

7. Verfahren nach mehreren der Ansprüche 1 bis 6,
**dadurch gekennzeichnet**, daß man ein unter 0.1 liegendes Gewichtsverhältnis zwischen Katalysator und Glu-

14

cose anwendet.

## Claims

1. A process for the production of gluconic acid or alkali metal salts thereof by oxidation of glucose with oxygen or an oxygen-containing gas in an aqueous alkaline solution in the presence of a supported catalyst containing a platinum group metal and bismuth,
characterized in that the reaction is carried out at a temperature of 20 to 80°C and at a pH value of 8.0 to 11 - maintained by continuous addition of alkali metal hydroxide - to an optimal degree of conversion - measurable by intermediate analysis - on a supported catalyst of which the quantity bears a ratio by weight to the glucose of at most 0.2, which has an active phase of platinum, palladium and bismuth applied to active carbon as support and which contains 0.1 to 10% by weight of the platinum- and palladium-containing noble metal component and 0.01 to 20% by weight bismuth for a ratio by weight of platinum to palladium of 1:99 to 70:30, preferably 15:85 to 50:50 and, more preferably, 15:85 to 25:75 and, after separation of the catalyst, the alkali metal gluconate formed is optionally converted into the free acid.

2. A process as claimed in claim 1,
characterized in that the reaction is carried out under pressures of the gaseous oxidizing agent from normal pressure to 3.0 bar.

3. A process as claimed in claim 1 or 2,
characterized in that the glucose is used in the form of a 10 to 20% by weight aqueous solution.

4. A process as claimed in any of claims 1 to 3,
characterized in that a reaction temperature of 30 to 60°C is maintained.

5. A process as claimed in any of claims 1 to 4,
characterized in that it is carried out at a pH value of 9 to 11.

6. A process as claimed in any of claims 1 to 5,
characterized in that powder-form active carbon is used as the catalyst support.

7. A process as claimed in several of claims 1 to 6,
characterized in that a ratio by weight of catalyst to glucose below 0.1 is used.

## Revendications

1. Procédé d'obtention d'acide gluconique ou de ses sels de métal alcalin par oxydation du glucose par l'oxygène ou par un gaz renfermant de l'oxygène en solution aqueuse alcaline en présence d'un catalyseur support contenant un métal du groupe du Platine et du Bismuth,
caractérisé en ce que l'on effectue la réaction à une température de 20 à 80°C, et à une valeur de pH de 8,0 à 11 maintenue par addition constante d'hydroxyde de métal alcalin jusqu'à obtention d'un degré optimal de conversion reconnaissable par analyse intermédiaire, sur un catalyseur sur support dont la quantité par rapport au glucose représente un rapport pondéral d'au plus 0,2, possède une phase active constituée de Platine, de Palladium et de Bismuth appliquée sur du charbon actif comme support et contient de 0,1 à 10 % en poids de composants en métaux nobles renfermant du Platine et du Palladium et de 0,01 à 20 % en poids de Bismuth pour un rapport pondéral entre le Platine et le Palladium de 1 : 99 à 70 : 30, de préférence de 15 : 85 à 50 : 50 et en particulier de 15 : 85 à 25 : 75, et après séparation du catalyseur le gluconate de métal alcalin formé est transformé éventuellement en acide libre.

2. Procédé selon la revendication 1,
caractérisé en ce que l'on effectue la réaction à des pressions de l'agent d'oxydation gazeux allant de la pression normale à 3,0 bars.

3. Procédé selon la revendication 1 ou la revendication 2,
caractérisé en ce que l'on met en oeuvre le glucose sous forme d'une solution aqueuse à 10 - 20 % en poids.

4. Procédé selon l'une des revendications 1 à 3,
caractérisé en ce que l'on maintient une température de réaction de 30 à 60°C.

5. Procédé selon l'une des revendications 1 à 4,
caractérisé en ce que l'on travaille à une valeur de pH allant de 9 à 11.

6. Procédé selon l'une des revendications 1 à 5,
caractérisé en ce que l'on met en oeuvre comme support de catalyseur du charbon actif pulvérulent.

7. Procédé selon plusieurs des revendications 1 à 6,
caractérisé en ce que l'on utilise un rapport pondéral qui se situe en-dessous de 0,1 entre le catalyseur et le glucose.